# EUROPEAN PATENT SPECIFICATION

(11) **EP 2 101 787 B1**
(45) Date of publication and mention of the grant of the patent: **18.09.2013**
(21) Application number: 07846446.8
(22) Date of filing: 11.12.2007
(51) Int. Cl.: A61K 31/665, A61P 11/00, A61P 31/04

(54) **METHODS FOR TREATING CYSTIC FIBROSIS OR PNEUMONIA WITH BACTERIAL INFECTION VIA PULMONARY ADMINISTRATION OF FOSFOMYCIN**
VERFAHREN ZUR BEHANDLUNG VON ZYSTISCHER FIBROSE ODER PNEUMONIE MIT BAKTERIELLER INFEKTION DURCH PULMONALE VERABREICHUNG VON FOSFOMYCIN
PROCÉDÉS DE TRAITEMENT DE LA MUCOVISCIDOSE OU DE LA PNEUMONIE AVEC INFECTION BACTÉRIENNE PAR ADMINISTRATION PULMONAIRE DE FOSFOMYCINE

(30) Priority: 11.12.2006 US 874081 P
(43) Date of publication of application: 23.09.2009
(73) Proprietor: Drugrecure ApS, 2970 Hørsholm (DK)
(72) Inventor: FIALA, Kaare, 2000 Frederiksberg (DK)
(74) Representative: Høiberg, Susanne
(86) International application number: PCT/DK2007/050184
(87) International publication number: WO 2008/071197

(56) References cited:
- WO-A-2005/110022
- MIRAKHUR A ET AL: "Fosfomycin therapy for multiresistant Pseudomonas aeruginosa in cystic fibrosis" JOURNAL OF CYSTIC FIBROSIS, ELSEVIER, vol. 2, 2003, pages 19-24, XP002430613 ISSN: 1569-1993

## Description

### Field of the Invention

The present invention provides a composition for use in a method for treating a bacterial infection in a subject suffering from cystic fibrosis or pneumonia or bacterial colonization of the airways by administering to the subject an effective amount of fosfomycin via pulmonary administration.

### Background of the Invention

Fosfomycin is a broad spectrum antibiotic that, for example, has been approved as a single-dose therapy for uncomplicated urinary tract infections. Fosfomycin is the international nonproprietary name (INN) corresponding to the compound with the chemical name (2*R*,3*S*)-3-methyloxiran-2-yl]phosphonic acid, which has the following formula:

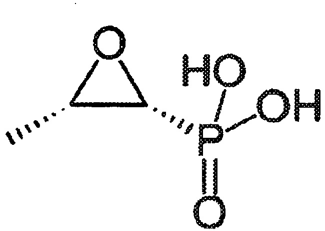

Fosfomycin was isolated from a Streptomyces species in 1970 and has bactericidal activity against both Gram-negative and Gram-positive bacteria.

Fosfomycin is a phosphoenolpyruvate analogue produced by Streptomyces that irreversibly inhibits enolpyruvate transferase (MurA), which prevents the formation of N-acetylmuramic acid, an essential element of the peptidoglycan cell wall. Fosfomycin tromethamine/trometamol and fosfomycin calcium are generally administered orally as a powder dissolved in water. Disodium fosfomycin has also been administered intravenously and intramuscularly for a variety of infections.

Aminoglycosides such as TOBI or tobramycin have been suggested to be useful in treatment infections in patients with cystic fibrosis. See U.S. Patent 5,840,702. However, multiple treatments with antibiotics such as TOB I have led to bacteria-resistant strains to these antibiotics. WO 2005/110022 discloses aerosol formulations comprising fosfomycin and tobramycin for treatment of infections in the respiratory tract for example infections in connection with cystic fibrosis and pneumonia.

### Summary of the Invention

Tobramycin is an often used antibiotic in early phases of infection. There may, however, develop widespread tobramycin resistance and there is thus a need for a new antiobiotic with a different mechanism of action in order to circumvent resistance to tobramycin. Inhalation of fosfomycin is a more effective treatment of infections in the lung than treatment of patients by systemic administration of fosfomycin and thus inhalation will be a more effective treatment modality. Local administration of fosfomycin further diminishes the risk of systemic side-effects.

The present invention provides a composition comprising fosfomycin for use in a method for treating a bacterial infection or bacterial airway colonization in a subject in need thereof, wherein fosfomycin is administered in a dose of 1 mg/kg bodyweight to 100 mg/kg bodyweight via local pulmonary administration, and wherein the subject is human and fosfomycin is the only active pharmaceutical ingredient comprised in said composition.

In one aspect, the present invention relates to a method for treating a bacterial infection or bacterial airway colonization in a subject in need which comprises administering to the subject via pulmonary administration an effective amount of fosfomycin. The method of the present invention is particularly useful in treating a bacterial infection and/or a bacterial airway colonization in a subject suffering from pulmonary disease including but not limited to pneumonia or cystic fibrosis with bacterial infection and/or lung colonization while minimizing the systemic adverse reactions and/or fosfomycin related organ toxicity associated with fosfomycin administration.

Another aspect of the present invention relates to the use of fosfomycin in the manufacture of a medicament for local pulmonary administration to treat a bacterial infection or bacterial airway colonization in a subject in need thereof. Fosfomycin via pulmonary administration is particularly useful in treating a bacterial infection or bacterial airway colonization in a subject suffering from pulmonary condition including but not limited to bacterial pneumonia, cystic fibrosis with bacterial infection and/or bacterial lung and/or airway colonization while minimizing systemic adverse reactions and/or fosfomycin related organ toxicity associated with systemic fosfomycin administration. The medicament may be manufactured with fosfomycin alone.

### Detailed Description of the Invention

### Fosfomycin

The present invention relates to administration via pulmonary administration to a human subject, inclusive of both adults and children, fosfomycin, in an amount effective to treat a bacterial infection in the subject. Administration of an effective amount of fosfomycin via pulmonary administration is particularly useful in treating a bacterial infection in a subject suffering from pneumonia or cystic fibrosis with bacterial infection and/or bacterial colonization of the airway and/or lung.

Fosfomycin for use in the present invention may for example be available through various commercial vendors. Fosfomycin of this application is meant to be inclusive of fosfomycin tromethamine, fosfomycin calcium, phosphonomycin and disodium fosfomycin.

### Medical Indications

For purposes of the present invention by "treating" a bacterial infection it is meant to include bacteriostatic as well as bacteriocidal activities including, but not limited to deterring further growth of bacteria and/or killing bacteria infecting the lungs and/or airways of a subject and/or preventing and/or inhibiting and/or deterring infection by bacteria in the lungs and/or airways of a subject.

Administration of an effective amount of fosfomycin via pulmonary administration is particularly useful in alleviating symptoms and/or treating subjects suffering from conditions including, but not limited pneumonia and cystic fibrosis with bacterial, and/or colonization or the airways and/or lung parenchyma.

The spectrum of diseases encompasses the following pulmonary conditions and or infections like Bronchitis, Cystic fibrosis, Bronchiectasis, Diffuse panbronchiolitis, Bronchiolitis, Bronchiolitis obliterans, Bronchiolitis obliterans organizing pneumonia (BOOP), Pneumonia of any cause, including but not limited to Community acquired pneumonia, Nosocomial pneumonia and Ventilator associated pneumonia (VAP).In preferred embodiments of the invention the pulmonary conditions may be Cystic fibrosis and Pneumonia of any cause, including but not limited to Community acquired pneumonia, Nosocomial pneumonia and Ventilator associated pneumonia (VAP).

Infections may for example be an infection by bacteria. For example infection by one or more bacteria selected from the group consisting of Achromobacter xylosoxidans, Acinetobacter calcoaceticus, preferably A. anitratus, A. haemolyticus, A. alcaligenes, and A. Iwoffii, Actinomyces israelii, Aeromonas hydrophilia, Alcaligenes species, preferably A. faecalis, A. odorans and A. denitrificans, Arizona hinshawii, Bacillus anthracis, Bacillus cereus, Bacteroides fragilis, Bacteroides melaninogenicus, Bordetella pertussis, Borrelia burgdorferi, Borrelia recurrentis, Brucella species, preferably B. abortus, B. suis, B. melitensis and B. canis, Calymmatobacterium granulomatis, Campylobacter fetus ssp. intestinalis, Campylobacter fetus ssp. jejuni, Chlamydia species, preferably C. psittaci and C. trachomatis, Chromobacterium violaceum, Citrobacter species, preferably C. freundii and C. diversus, Clostridium botulinum, Clostridium perfringens, Clostridium difficile, Clostridium tetani, Corynebacterium diphtheriae, Corynebacterium, preferably C. ulcerans, C. haemolyticum and C. pseudotuberculosis, Coxiella burnetii, Edwardsiella tarda, Eikenella corrodens, Enterobacter, preferably E. cloacae, E. aerogenes, E. hafniae (also named Hafnia alvei) and E. agglomerans, Erysipelothrix rhusiopathiae, Escherichia coli, Flavobacterium meningosepticum, Francisella tularensis, Fusobacterium nucleatum, Gardnerella vaginalis, Haemophilus ducreyi, Haemophilus influenzae, Helicobacter species, Klebsiella species, preferably K. pneumoniae, K. ozaenae og K. rhinoscleromatis, Legionella species, Leptospira interrogans, Listeria monocytogenes, Moraxella species, preferably M. lacunata and M. osloensis, Mycoplasma species, preferably M. pneumoniae, Neisseria gonorrhoeae, Neisseria meningitidis, Pasterurella haemolytica, Pasteurella multocida, Peptococcus magnus, Plesiomonas shigelloides, Pneumococci, Proteus species, preferably P. mirabilis, P. vulgaris, P. rettgeri and P. morganii (also named Providencia rettgeri and Morganella morganii respectively), Providencia species, preferably P. alcalifaciens, P. stuartii and P. rettgeri (also named Proteus rettgeri), Pseudomonas aeruginosa, Pseudomonas mallei, Pseudomonas pseudomallei, Rickettsia, Rochalimaia henselae, Salmonella species, preferably S. enteridis, S. typhi and S. derby, and most preferably Salmonella species of the type Salmonella DT104, Serratia species, preferably S. marcescens, Shigella dysenteriae, S. flexneri, S. boydii and S. sonnei, Spirillum minor, Staphylococcus aureus, Staphylococcus epidermidis, Staphylococcus saprophyticus, Streptobacillus moniliformis, Streptococcus, preferably S. faecalis, S. faecium and S. durans, Streptococcus agalactiae, Streptococcus pneumoniae, Streptococcus pyogenes, Treponema carateum, Treponeam pallidum, Treponema pertenue, preferably T. pallidum, Ureaplasma urealyticum, Vibrio cholerae, Vibrio parahaemolyticus, Yersinia enterocolitica, and Yersinia pestis.

### Administration

Methods of pulmonary administration include, but are not limited to, spraying, lavage, inhalation, flushing or installation, using as fluid a physiologically acceptable composition in which fosfomycin have been dissolved. When used herein the terms "intratracheal, intrabronchial or intraalveolar administration" include all forms of such administration whereby fosfomycin is applied into the trachea, the bronchi or the alveoli, respectively, whether by the instillation of a solution of fosfomycin, by applying fosfomycin in a powder form, or by allowing fosfomycin to reach the relevant part of the airway by inhalation of fosfomycin as an aerosolized or nebulized solution or suspension or inhaled powder or gel, with or without added stabilizers or other excipients.

It is expected that pulmonary administration of fosfomycin in accordance with the present invention will minimize systemic adverse reactions associated with fosfomycin administration. Methods of intrabronchial/alveolar administration include, but are not limited to, bronchoalveolar lavage (BAL) according to methods well known to those skilled in the art, using as a lavage fluid a physiologically acceptable composition in which the fosfomycin has been dissolved or indeed by any other effective form of intrabronchial administration including the use of nebulized powders containing fosfomycin in dry form, with or without excipients, or the direct application of fosfomycin, in solution or powder form during bronchoscopy. Methods for intratracheal administration include, but are not limited to, blind tracheal washing with a similar solution of dissolved fosfomycin, or the inhalation of nebulized fluid droplets containing dissolved fosfomycin obtained by use of any nebulizing apparatus adequate for this purpose.

In one embodiment, intratracheal, intrabronchial or intraalveolar administration and/or administration to small airways such as bronchioli does not necessarily include inhalation of the product but the instillation or application of a solution of fosfomycin or a powder containing fosfomycin into the trachea or lower airways.

Methods of intrabronchial/alveolar administration include, but are not limited to, bronchoalveolar lavage (BAL) according to methods well known to those skilled in the art, using as a lavage fluid a physiologically acceptable composition in which fosfomycin been dissolved or indeed by any other effective form of intrabronchial administration including the use of inhaled powders containing fosfomycin in dry form, with or without excipients, or the direct application of fosfomycin, in solution or suspension or powder form during bronchoscopy. Methods for intratracheal administration include, but are not limited to, blind tracheal washing with a similar solution of dissolved fosfomycin or a fosfomycin suspension, or the inhalation of nebulized fluid droplets containing dissolved fosfomycin or a fosfomycin suspension obtained by use of any nebulizing apparatus adequate for this purpose.

In another embodiment, intratracheal, intrabronchial or intraalveolar administration does not include inhalation of the product but the instillation or application of a solution of fosfomycin or a powder or a gel containing fosfomycin into the trachea or lower airways.

Preferred concentrations for a solution comprising fosfomycin and/or functional homologues or variants of fosfomycin are in the range of 0.1 µg to 10000 µg active ingredient per ml solution. The suitable concentrations are often in the range of from 0.1 µg to 5000 µg per ml solution, such as in the range of from about 0.1 µg to 3000 µg per ml solution, and especially in the range of from about 0.1 µg to 1000 µg per ml solution, such as in the range of from about 0.1 µg to 250 µg per ml solution. A preferred concentration would be from about 0.1 to about 5.0 mg, preferably from about 0.3 mg to about 3.0 mg, such as from about 0.5 to about 1.5 mg and especially in the range from 0.8 to 1.0 mg per ml solution.

Other preferred methods of administration may include using the following devices:
1. Pressurized nebulizers, e.g. jet nebulizers, using compressed air/oxygen mixture
2. Ultrasonic nebulizers
3. Electronic micropump nebulizers (e.g. Aeroneb Professional Nebulizer)
4. Metered dose inhaler (MDI)
5. Dry powder inhaler systems (DPI)

The aerosol may be delivered by via a) facemasks or b) via endotracheal tubes in intubated patients during mechanical ventilation (device 1, 2 and 3). The devices 4 and 5 can also be used by the patient without assistance provided that the patient is able to self-activate the aerosol device.

### Pharmaceutical composition

Pharmaceutical compositions or formulations for use in the present invention include fosfomycin in combination with, preferably dissolved in, a pharmaceutically acceptable carrier, preferably an aqueous carrier or diluent, or carried to the lower airways as a pegylated preparation or as a liposomal or nanoparticle preparation administered as an aerosol via inhalation, or as a lavage fluid administered via a bronchoscope as a bronchoalveloar lavage or as a blind intratracheal wash or lavage. A variety of aqueous carriers may be used, including, but not limited to 0.9% saline, buffered saline, physiologically compatible buffers and the like. The compositions may be sterilized by conventional techniques well known to those skilled in the art. The resulting aqueous solutions may be packaged for use or filtered under aseptic conditions and freeze-dried, the freeze-dried preparation being dissolved in a sterile aqueous solution prior to administration.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

Fosfomycin may be administered alone.

Pharmaceutical compositions or formulations for use in the present invention include fosfomycin in combination with, preferably dissolved in, a pharmaceutically acceptable carrier, preferably an aqueous carrier or diluent, or carried to the lower airways as a pegylated preparation or as a liposomal or nanoparticle preparation administered as an aerosol via inhalation, or as a lavage fluid administered via a bronchoscope as a bronchoalveloar lavage or as a blind intratracheal wash or lavage. A variety of aqueous carriers may be used, including, but not limited to 0.9% saline, buffered saline, physiologically compatible buffers and the like. The compositions may be sterilized by conventional techniques well known to those skilled in the art. The resulting aqueous solutions may be packaged for use or filtered under aseptic conditions and freeze-dried, the freeze-dried preparation being dissolved in a sterile aqueous solution prior to administration

In one embodiment a freeze-dried fosfomycin preparation may be pre-packaged for example in single dose units. In an even more preferred embodiment the single dose unit is adjusted to the patient.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

The formulations may contain pharmaceutically acceptable carriers and excipients including microspheres, microcapsules, nanoparticles or the like. Preferably liposomes are in not used in the fosfomycin formulations for the present invention.
In some cases, it will be advantageous to include a compound, which promotes delivery of the active substance to its target.

### Dose

By "effective amount" of fosfomycin, it is meant a dose, which, when administered via pulmonary administration, achieves a local pulmonary concentration of fosfomycin in the subject's airways and/or lung parenchyma which has a bacteriostatic effect and/ or a bacteriocidal effect on a bacterial infection and/or colonization. In a preferred embodiment, the dose administered achieves a high local pulmonary fosfomycin concentration several fold above the minimal inhibitory concentration (MIC) of typically about 10 µg/ml for antibiotic-resistant bacteria and typically about 1 µg/ml for non-antibiotic resistant bacteria. Doses expected to provide an effective amount of fosfomycin are in the range of about 50 mg to 5000 mg, more preferably about 200 mg to about 4 g, yet more preferably about 400 mg to about 3.5 g, even more preferably about 500 mg to about 3 g, yet more preferably from about 600 mg to about 3.5 mg, preferably from about 1 to 2 g administered via inhalation, preferably via a nebulizer connected to a facemask or to an endotracheal tube or via bronchoalveolar lavage (BAL), one to three times daily.

Fosfomycin may be administered from for example one to seven days a week, preferably from one to six days a week, more preferably from one to five days a week, yet more preferably from 2 to four days a week or even more preferably from two to three days a week.

Duration of dosing will typically range from 1 day to about 4 months, such as 2 days to about 3 months, for example in the range of 1-2 days to 2 months, such as in the range of 1-2 days to 1 month.

The preparations are administered in a manner compatible with the dosage formulation, and in such amount as will be therapeutically effective. The quantity to be administered depends on the subject to be treated, including, e.g. the weight and age of the subject, the disease to be treated and the stage of disease. Suitable dosage ranges are per kilo body weight expected to provide an effective amount of fosfomycin are of the order of 0.5 mg to 150 mg per kilo body weight, such as in the range of from about 0.75 mg to 125 mg per kilo body weight, and especially in the range of from about 1 mg to 100 mg per kilo body weight, preferably in the range of 5 mg to 90 mg, for example in the range of 10 mg to 80 mg, such as in the range of from about 15 mg to 80 mg per kilo body weight, and preferably in the range of from about 25 mg to 75 mg per kilo body weight administered between one and seven times daily, such as between two and six times daily, for example between three and five times daily, preferably around three to four times daily

### Medical packaging

The compounds used in the invention may be administered alone or in combination with pharmaceutically acceptable carriers or excipients, in either single or multiple doses. The formulations may conveniently be presented in unit dosage form by methods known to those skilled in the art.

It is preferred that the compounds according to the invention are provided in a kit. Such a kit typically contains an active compound in dosage forms for administration. A dosage form contains a sufficient amount of active compound such that a desirable effect can be obtained when administered to a subject.

Thus, it is preferred that the medical packaging comprises an amount of dosage units corresponding to the relevant dosage regimen. Accordingly, in one embodiment, the medical packaging comprises a pharmaceutical composition comprising a compound as defined above or a pharmaceutically acceptable salt thereof and pharmaceutically acceptable carriers, vehicles and/or excipients, said packaging comprising from 1 to 7 dosage units, thereby having dosage units for one or more days, or from 7° to 21 dosage units, or multiples thereof, thereby having dosage units for one week of administration or several weeks of administration.

The dosage units can be as defined above. The medical packaging may be in any suitable form for intratracheal, intrabronchial or intraalveolar administration. In a preferred embodiment the packaging is in the form of a vial, ampule, tube, blister pack, cartridge or capsule.

When the medical packaging comprises more than one dosage unit, it is preferred that the medical packaging is provided with a mechanism to adjust each administration to one dosage unit only.

Preferably, a kit contains instructions indicating the use of the dosage form to achieve a desirable affect and the amount of dosage form to be taken over a specified time period. Accordingly, in one embodiment the medical packaging comprises instructions for administering the pharmaceutical composition.

Even more preferably a freeze-dried fosfomycin preparation may be pre-packaged for example in single dose units. In an even more preferred embodiment the single dose unit is adjusted to the patient.

### Examples

### Example 1

A patient with severe community acquired pneumonia with infiltrates on chest radiograph as defined by (Ewig S et al., (1998)) is treated with broad spectrum antibiotics intravenously. Due to the patients lack of response to treatment of IV antibiotic therapy and oxygen supplementation, the condition deteriorates with systemic symptoms including shock and organ dysfunction and need of a high inspired oxygen fraction and later mechanical ventilation. The therapy is supplemented with inhalation of fosfomycin in a dose of 25 - 75mg/kg typically two to four times per day. The inhalation is mediated via a nebulizer, i.e. a jet driven nebulizer, ultrasound nebulizer and/or a micropump nebulizer using a vibrating meshed disk. The inhalation therapy of fosfomycin continues for 5 to 10 days or until an objective positive treatment response is obtained, i.e. defervescence of signs of infections, e.g. reduced body temperature, normalization of leukocyte count, C-reactive protein (CRP) and/or procalcitonin test (PCT) and reduction of signs of shock, respiratory failure and improvement in organ dysfunction.

### Example 2

A critically ill patient undergoing mechanical ventilation for 3 days shows signs and symptoms of ventilator acquired pneumonia (VAP). The patient is treated with a combination of IV antibiotics and supplemented with inhalation of fosfomycin in a dose of 25 - 75mg/kg typically two to four times per day mediated via a nebulizer, i.e. a jet driven nebulizer, ultrasound nebulizer and or a micropump nebulizer using a vibrating meshed disk. The inhalation of fosfomycin continues for 5 to 10 days or until an objective positive treatment response is obtained, i.e. defervescence of signs of infections, e.g. reduced body temperature, normalization of leukocyte count, C-reactive protein(CRP) and or procalcitonin test (PCT) and reduction of signs of shock, respiratory failure and improvement in organ dysfunction.

### Example 3

A patient with cystic fibrosis has signs and symptoms of colonization of the lungs. In spite of former treatment with IV antibiotics and eventually treated with inhaled antibiotics like tobramycin but not fosfomycin, is treated with inhalation of fosfomycin in a dose of 25 - 75mg/kg typically two to four times per day mediated via a nebulizer, i.e. a jet driven nebulizer, ultrasound nebulizer and or a micropump nebulizer using a vibrating meshed disk. The inhalation of fosfomycin continues for 5 to 21 days or until an objective positive treatment response is obtained, i.e. defervescence of signs of infections, e.g. reduced body temperature, normalization of leukocyte count, C - reactive protein (CRP) and or procalcitonin test (PCT) and reduction of signs of respiratory failure like dyspnoea respiratory rate.

### Example 4

A patient with pneumonia and or airway colonization with methicillin resistant Staphylococcus aureus (MRSA) is treated with inhalation of fosfomycin in a dose of 10 - 75mg/kg typically two to four times per day mediated via a nebulizer, i.e. a jet driven nebulizer, ultrasound nebulizer and or a micropump nebulizer using a vibrating meshed disk. The inhalation of fosfomycin continues for 5 to 21 days or until a culture negative sputum is obtained or an objective positive treatment response is obtained, i.e. defervescence of signs of infections, e.g. reduced body temperature, normalization of leukocyte count, C-reactive protein (CRP) and or procalcitonin test (PCT). The inhalation of fosfomycin may be combined with intravenous antibiotics with fosfomycin or other MRSA sensitive antibiotics.

### References

Ewig S, Ruiz M, Mensa J, Marcos MA, Martinez JA, Arancibia F, Niederman MS, Torres A. Severe community-acquired pneumonia. Assessment of severity criteria. Am J Respir Crit Care Med. 1998 Oct;158(4):1102-8

## Claims

1. A composition comprising fosfomycin for use in the treatment of a bacterial infection and/or bacterial colonization of the lungs and/or airways of a human subject, wherein fosfomycin is administered in a dose of from 1 mg/kg bodyweight to 100 mg/kg bodyweight via local pulmonary administration, and wherein fosfomycin is the only active pharmaceutical ingredient comprised in said composition.

2. The composition for use according to claim 1, wherein one dose of fosfomycin is between 25 mg/kg bodyweight to 75 mg/kg bodyweight.

3. The composition for use according to any of the preceding claims, wherein the dose of fosfomycin is administered two to six times daily.

4. The composition for use according to any of the preceding claims, wherein the dose of fosfomycin is administered three to four times daily.

5. The composition for use according to any of the preceding claims, wherein fosfomycin is administered by intratracheal, intrabronchial or intraalveolar administration.

6. The composition for use according to any of the preceding claims, wherein the subject is suffering from pneumonia of any cause including community acquired pneumonia, nosocomial pneumonia and ventilator associated pneumonia (VAP); bronchitis, diffuse panbronchiolitis, bronchiolitis, bronchiolitis obliterans, bronchiolitis obliterans organizing pneumonia (BOOP), bronchiectasis or cystic fibrosis, each with bacterial infection and/or colonization of the lung and/or airways.

7. The composition for use according to any of the preceding claims, wherein the subject is administered a solution of fosfomycin via bronchoalveolar lavage.

8. The composition for use according to any of the preceding claims, wherein the subject is administered a solution of fosfomycin via blind tracheal washing.

9. The composition for use according to any of the preceding claims, wherein the subject is administered a nebulized solution of fosfomycin.

10. The composition for use according to any of the preceding claims, wherein the subject is administered an aerosol or powder form of fosfomycin.

11. The composition for use according to any of the preceding claims, wherein the subject is administered a pegylated or nanoparticle prepared form of fosfomycin.

12. The composition for use according to any of the preceding claims, wherein the subject is administered fosfomycin during bronchoscopy.

## Patentansprüche

1. Zusammensetzung, umfassend Fosfomycin zur Verwendung bei der Behandlung einer bakteriellen Infektion und/oder bakteriellen Besiedlung der Lunge und/oder Luftwege eines menschlichen Subjekts, worin Fosfomycin mittels lokal pulmonärer Verabreichung in einer Dosis von 1 mg/kg Körpergewicht bis 100 mg/kg Körpergewicht verabreicht wird, und worin Fosfomycin den ausschließlichen aktiven pharmazeutischen Bestandteil darstellt, der in der Zusammensetzung enthalten ist.

2. Zusammensetzung zur Verwendung nach Anspruch 1, worin eine Dosis an Fosfomycin zwischen 25 mg/kg Körpergewicht und 75 mg/kg Körpergewicht beträgt.

3. Zusammensetzung zur Verwendung nach einem der vorstehenden Ansprüche, worin die Dosis an Fosfomycin zwei- bis sechsmal täglich verabreicht wird.

4. Zusammensetzung zur Verwendung nach einem der vorstehenden Ansprüche, worin die Dosis an Fosfomycin drei- bis viermal täglich verabreicht wird.

5. Zusammensetzung zur Verwendung nach einem der vorstehenden Ansprüche, worin Fosfomycin mittels intratrachealer, intrabronchialer oder intraalveolärer Verabreichung verabreicht wird.

6. Zusammensetzung zur Verwendung nach einem der vorstehenden Ansprüche, worin das Subjekt an Pneumonie irgendeiner Ursache erkrankt ist, einschließlich ambulant erworbener Pneumonie, nosokomialer Pneumonie und mit Beatmung zusammenhängender Pneumonie (VAP); Bronchitis, diffuser Panbronchiolitits, Bronchiolitis, Bronchiolitis obliterans, Bronchiolitis obliterans organisierende Pneumonie (BOOP), Bronchiektasie oder zystischer Fibrose, jede mit bakterieller Infektion und/oder Besiedelung der Lunge und/oder Luftwege.

7. Zusammensetzung zur Verwendung nach einem der vorstehenden Ansprüche, worin dem Subjekt eine Fosfomycin-Lösung mittels bronchoalveolärer Lavage verabreicht wird.

8. Zusammensetzung zur Verwendung nach einem der vorstehenden Ansprüche, worin dem Subjekt eine Fosfomycin-Lösung mittels blinder Bronchiallavage verabreicht wird.

9. Zusammensetzung zur Verwendung nach einem der vorstehenden Ansprüche, worin dem Subjekt eine zerstäubte Fosfomycin-Lösung verabreicht wird.

10. Zusammensetzung zur Verwendung nach einem der vorstehenden Ansprüche, worin dem Subjekt Fosfomycin in Aerosol- oder Pulverform verabreicht wird.

11. Zusammensetzung zur Verwendung nach einem der vorstehenden Ansprüche, worin dem Subjekt Fosfomycin in PEGylierter oder in einer durch Nanopartikel hergestellten Form verabreicht wird.

12. Zusammensetzung zur Verwendung nach einem der vorstehenden Ansprüche, worin dem Subjekt Fosfomycin während einer Bronchoskopie verabreicht wird.

## Revendications

1. Composition comprenant de la fosfomycine pour une utilisation dans le traitement d'une infection bactérienne et/ou d'une colonisation bactérienne des poumons et/ou des voies respiratoires d'un sujet humain, dans laquelle la fosfomycine est administrée dans une dose de 1 mg/kg de poids corporel à 100 mg/kg de poids corporel par l'intermédiaire d'une administration pulmonaire locale, et dans laquelle la fosfomycine est le seul composant pharmaceutique actif compris dans ladite composition.

2. Composition pour une utilisation selon la revendication 1, dans laquelle une dose de fosfomycine se situe entre 25 mg/kg de poids corporel à 75 mg/kg de poids corporel.

3. Composition pour une utilisation selon l'une quelconque des revendications précédentes, dans laquelle la dose de fosfomycine est administrée deux à six fois par jour

4. Composition pour une utilisation selon l'une quelconque des revendications précédentes, dans laquelle la dose de fosfomycine est administrée trois à quatre fois par jour.

5. Composition pour une utilisation selon l'une quelconque des revendications précédentes, dans laquelle la fosfomycine est administrée par administration intratrachéale, intrabronchique ou intra-alvéolaire.

6. Composition pour une utilisation selon l'une quelconque des revendications précédentes, dans laquelle le sujet souffre de pneumonie d'une cause quelconque y compris une pneumonie communautaire, une pneumonie nosocomiale et une pneumonie associée aux ventilateurs (PAV) ; une bronchite, une panbronchiolite diffuse, une bronchiolite, une bronchiolite oblitérante, une bronchiolite oblitérante avec pneumonie organisée (BOOP), une bronchiectasie ou une mucoviscidose, chacune avec infection bactérienne et/ou colonisation du poumon et/ou des voies respiratoires.

7. Composition pour une utilisation selon l'une quelconque des revendications précédentes, dans laquelle il est administré au sujet une solution de fosfomycine par lavage bronchoalvéolaire.

8. Composition pour une utilisation selon l'une quelconque des revendications précédentes, dans laquelle il est administré au sujet une solution de fosfomycine par lavage trachéal à l'aveugle.

9. Composition pour une utilisation selon l'une quelconque des revendications précédentes, dans laquelle il est administré au sujet une solution nébulisée de fosfomycine.

10. Composition pour une utilisation selon l'une quelconque des revendications précédentes, dans laquelle il est administré au sujet une forme d'aérosol ou de poudre de la fosfomycine.

11. Composition pour une utilisation selon l'une quelconque des revendications précédentes, dans laquelle il est administré au sujet une forme préparée pégylée ou nanoparticulaire de la fosfomycine.

12. Composition pour une utilisation selon l'une quelconque des revendications précédentes, dans laquelle il est administré au sujet de la fosfomycine durant une bronchoscopie.
